# EUROPÄISCHE PATENTSCHRIFT

(11) **EP 0 516 252 B1**
(45) Veröffentlichungstag und Bekanntmachung des Hinweises auf die Patenterteilung: **30.07.1997**
(21) Anmeldenummer: 92250084.8
(22) Anmeldetag: 13.04.1992
(51) Int. Cl.: A61K 49/00, A61K 51/00, A61K 47/48, A61K 51/02

(54) **Nanokristalline magnetische Eisenoxid-Partikel zur Anwendung in Diagnostik und Therapie**
Monocrystalline particles of iron-oxide for application in medical diagnosis and therapy
Particules monocristallines d'oxyde ferrique pour application en diagnostique et thérapie

(30) Priorität: 28.05.1991 DE 4117782
(43) Veröffentlichungstag der Anmeldung: 02.12.1992
(73) Patentinhaber: INSTITUT FÜR DIAGNOSTIKFORSCHUNG GmbH AN DER FREIEN UNIVERSITÄT BERLIN, 14050 Berlin (DE)
(72) Erfinder: Kresse, Mayk, W-1000 Berlin 19 (DE); Lawaczeck, Rüdiger, Dr., W-1000 Berlin 27 (DE); Pfefferer, Detlef, W-1000 Berlin 44 (DE)
(74) Vertreter: Wablat, Wolfgang, Dr.Dr.

(56) Entgegenhaltungen:
- EP-A- 0 186 616
- EP-A- 0 423 002
- EP-A- 0 444 194
- WO-A-90/01295
- WO-A-91/15753
- WO-A-91/16080

## Beschreibung

Die Erfindung betrifft nanokristalline magnetische Partikel, bestehend aus einem magnetischen Eisenoxidkern aus Fe₃ O₄,γ-Fe₂ O₃ oder deren Mischungen und einer an diesen Kern chemisorbierten Hülle, sowie deren kolloid-disperse wäßrige Lösung, Verfahren zur Herstellung dieser Partikel sowie ihre Anwendung in der medizinischen Diagnostik und Therapie.

Magnetische Substanzen haben als Kontrastmittel für die Kernspintomographie Eingang in die medizinische Anwendung gefunden. Als erstes zugelassenes Präparat ist hier ein GdDTPA-Komplex (Magnevist®) zu nennen. Für die Leberdiagnostik haben sich magnetische Eisenoxid-Partikel als besonders wirkungsvoll herausgestellt. Sie befinden sich in der vorklinischen und z. T. klinischen Entwicklung. Es handelt sich dabei zumeist um ferrimagnetische Eisenoxide (z.B. Magnetite), Nanopartikel, die zur Ausbildung stabiler wäßriger Sole mit einer Hülle umgeben werden.

Neben der Anwendung in der Diagnostik spielen ferro-/ferrimagnetische Partikel bei der in vitro Separationstechnik und als "Thermoseeds" in der lokalen Hyperthermie eine zunehmende Rolle.

In der wissenschaftlichen und patentrechtlichen Literatur sind zahlreiche Magnetitherstellungen und Magnetitanwendungen beschrieben.

Hasegawa und Hokkoku (US 4,101,435) beschreiben einen Eisenoxid-Dextran-Komplex und seine Herstellung.

Rembaum (US 4,267,234) bezieht sich auf magnetische Polyglutaraldehyd-Mikropartikel, die durch eine Suspensionspolymerisation in Gegenwart magnetischer Partikel hergestellt werden. Widder und Senyei (US 4,247,406) stellen Mikropartikel aus einer Aminosäure-Polymermatrix her, in die magnetische Teilchen eingebettet sind. Einen ähnlichen Weg gehen Schröder und Mosbach (WO 83/01738) mit Nanopartikeln, bei denen eine kristalline Kohlenwasserstoff-Matrix das magnetische Material einschließt. Groman und Josephson (US 4,770,183) verwenden nicht-umhüllte und mit einer Polysaccharid- und/oder Proteinschicht umhüllte magnetische Metalloxidpartikel. Molday (US 4,452,773) hebt auf die Synthese ferromagnetischer Eisenoxid-Kerne mit einer Polysaccharidhülle ab. Er erhält ein stabiles Sol und kann über eine Perjodataktivierung Proteine an die Hülle aus Dextranen koppeln.

Gordon (US 4,731,239) beansprucht die Verwendung ferro-, para- und diamagnetischer Partikel aus Eisenhydroxid, Eisenoxid und Eisendextran für diagnostische Zwecke. In weiteren Patenten (US 4,767,611, 4,758,429, 4,735,796) wird auf die Diagnose und Therapie mit Hilfe der erwähnten Eisendextran oder Eisen-Transferrin-Dextran-Partikel und alternierender elektromagnetischer Felder abgestellt. Die Partikel können zum Zielort über Antigene, Antikörper, Enzyme oder prosthetische Gruppen geleitet werden. Gries et al. (EP 186616) verwenden magnetische Partikel aus einem Doppelmetall-oxid/-hydroxid und einem Komplexbildner aus Proteinen oder alkalibehandelten Sacchariden bzw. Polysacchariden.

Im Laufe der Jahre hat die Komplexität der Partikel zugenommen und die Zielrichtung ist spezifischer geworden. Ranney (EP-361960) verwendet polyatomare Chrom-Komplexe, die an einen biokompatiblen Ausscheidungsträger gebunden sind.

Als Träger werden aufgezählt: Kohlenwasserstoffe, Polysaccharide, Glycosaminoglycane und strukturanaloge synthetische Polymere.

Diese Cr₄S-Cluster sind mit dem Nachteil behaftet, daß die Anzahl der ungepaarten Elektronen auf 12 beschränkt ist.

Das Einsatzgebiet Diagnostik wird um therapeutische Aspekte (Hyperthermie, Chemotherapie) erweitert.

Menz et al.(WO 9001295) zielen auf einen speziellen zellulären Aufnahmemechanismus (Rezeptor vermittelte Endocytose) mit ihren superparamagnetischen Kontrastmitteln ab.
Sie verwenden magnetische Eisenoxid-Partikel, die mit einer Hülle aus Arabinogalactan pflanzlicher Herkunft umgeben sind, deren toxikologische Unbedenklichkeit bisher noch nicht bestätigt werden konnte.

Yudelson (WO 8911154) deponiert eine Hülle aus Gelatine und einer polymeren Säure (vorzugsweise Gummiarabicum) auf den superparamagnetischen Partikeln durch Koazervation und Vernetzung mit vorzugsweise Glutaraldehyd. Einen anderen Weg geht Pilgrim (Europäische Patentanmeldung Publikationsnummer 0284549) durch Ausbildung einer chemischen Bindung zwischen Magnetitoberfläche und synthetischen Stabilisatorpolymersubstanzen. Die reaktive Stabilisatorsubstanz wird über Phosphat-, Phosphonat- oder Carboxylatgruppen mit den superparamagnetischen Teilchen chemisch verbunden. An die Stabilisatorsubstanz aus einem Polyethylengerüst können gewebespezifische Bindungssubstanzen addiert werden.

In beiden Fällen werden Herstellung und magnetische Eigenschaften der superparamagnetischen Partikel beschrieben, ohne daß die medizinische Eignung und die Verträglichkeit der Partikel belegt werden.

Magnetische Materialien werden aufgrund ihres Verhaltens im magnetischen Feld als dia-, para- oder ferromagnetisch eingestuft. Dia- und Paramagnetismus sind atomare/molekulare Eigenschaften, sie rühren vom magnetischen Moment des Elektrons her. Der Diamagnetismus beruht auf der Orbitalbewegung der Elektronen, er wird durch das angelegte Magnetfeld induziert und die Magnetisierung ist dem äußeren Magnetfeld entgegengesetzt ausgerichtet. Paramagnetische Substanzen zeichnen sich durch ein oder mehrere ungepaarte Elektronen aus, die Magnetisierung ist parallel zum äußeren Feld orientiert. Paramagnetische Atome/Moleküle haben damit ein permanentes magnetisches Moment, ohne äußeres Feld sind diese magnetischen Momente nicht ausgerichtet. Die Suszeptibilität ist unabhängig vom angelegten Feld und umgekehrt proportional zur Temperatur (Curie-Gesetz)

In Festkörpern kann es zu einer starken Wechselwirkung zwischen benachbarten Atomen/Ionen kommen, so daß eine spontane Magnetisierung resultiert, man spricht allgemein von Ferromagnetismus mit der genaueren Klassifizierung in Ferro-,Ferri- und Antiferromagnetismus. Beispiele ferromagnetischer Materialien sind die Metalle Eisen, Kobalt, Nickel, viele Seltene Erden sowie deren Legierungen. Bei Verbindungen, die aus unterschiedlichen Atomsorten, aus Ionen unterschiedlicher Oxidationszahl oder aus Ionen auf unterschiedlichen Gitterplätzen aufgebaut sind, können die magnetischen Momente benachbarter Atome/Ionen antiparallel orientiert sein. Bei kompletter Aufhebung der spontanen Magnetisierung spricht man von Antiferromagnetismus, bei unvollständiger Aufhebung von Ferrimagnetismus. Ferrite mit Spinellstruktur zeigen ferrimagnetisches Verhalten. In allen drei Fällen bilden sich im Festkörper Domänen mit unterschiedlicher Orientierung der spontanen Magnetisierung aus. Ohne äußeres Feld sind die magnetischen Momente der Domänen regellos verteilt, nach außen ergibt sich kein magnetisches Gesamtmoment. Im äußeren Magnetfeld richten sich die magnetischen Momente der Domänen aus, ein Zustand, der auch nach Abschalten des äußeren Magnetfeldes im Permanentmagnet erhalten bleibt. Man spricht von Remanenz. Für Partikel, die kleiner als Domänengröße sind, wirkt die thermische Energie einer spontanen parallelen/antiparallelen Ausrichtung der einzelnen magnetischen Momente entgegen. Suspensionen solcher ferro-/ferrimagnetischer Partikel mit Dimensionen kleiner als 10 - 50 nm verhalten sich ähnlich wie paramagnetische Materialien, sie zeigen keine Remanenz und Hysterese aber Werte für die Suszeptibilität, die an die entsprechenden Werte der Festkörper herankommen; sie werden deshalb oft auch als superparamagnetische Partikel bezeichnet. Ferrite, die als parenterale MR-Kontrastmittel eingesetzt werden, fallen in diese Kategorie (z.B. Wolf et al., Magnetic Resonance Annual, New York, Raven Press 1985, 231-266; Saini et al. Radiology 1987, 162,217-222).

Das MR-Image und die Signale der MR-Spektroskopie kommen aufgrund einer Verknüpfung dreier physikalischer Parameter zustande: Dichte der entsprechenden Kernsorte (z.B. Protonen, ¹³ Kohlenstoff, ³¹ Phosphor), Spin-Gitter-Relaxationszeit T₁ und Spin-Spin-Relaxationszeit T₂. T₁ und T₁ gehen in komplizierter Weise in die Signalintensität ein, sie sind Funktionen der Magnetfeldstärke, der Temperatur, der Umorientierung und der Art der physikalischen Wechselwirkung der einzelnen Moleküle. Die Signalintensität nimmt proportional zur Dichte des Beobachtungskerns zu. Für Protonen lassen sich die Beobachtungskerne grob in Fett- und Wasserprotonen einteilen. Die Protonendichte läßt sich im physiologischen Bereich nur in geringem Maß z. B. durch Deuteriumoxid-Substitution beeinflussen. Dagegen können die Relaxationszeiten durch Zusätze paramagnetischer Moleküle/Ionen (Moleküle mit ungepaarten Elektronen, z.B. Spin-Sonden oder Ionen der Seltenen Erden) relativ leicht beeinflußt werden. Die zugrunde liegenden Relaxationsmechanismen, z. B. durch paramagnetische Ionen, sind relativ gut verstanden und schlagen sich in den einschlägigen Monographien und Lehrbüchern nieder (Solomon-Bloembergen Gleichung).

Komplizierter gestaltet sich die physikalische Beschreibung der Relaxationseffekte im Fall partikulärer Wirkstoffe, insbesondere wenn diese nicht homogen verteilt vorliegen. Bei ferri-/ferromagnetischen Eisenoxid-Partikeln überwiegt der Einfluß auf die T₂-Zeit. Entsprechend der Konzentration der Eisenoxid-Partikel und der gewählten Pulssequenzen kann eine Verstärkung der Signale bis hin zur kompletten Signalauslöschung resultieren. Üblicherweise werden die Relaxationszeit-verkürzenden Eigenschaften der MR-Kontrastmittel durch die Relaxivität ausgedrückt. Gute - molekulargelöste - paramagnetische Substanzen zeigen bei 0.47 T in Wasser und 40°C Werte von ungefähr 4 l/mmol· s (GdDTPA) für die T₁ und T₂ Relaxivität. Dispersionen von superparamagnetischem Magnetit bzw. Maghemit liegen bei 20 - 40 für die T₁ und bei ungefähr 200 für die T₂-Relaxivität. Größe, Umhüllung, Anteil von γ-Fe₂ O₃ und Grad der Ausheilung kristalliner Fehlstellen tragen zu dem individuellen Wert bei.

Reine magnetische Eisenoxid-Partikel, die als Diagnostika in der Kernspintomographie in Frage kämen, aggregieren in wäßriger Lösung bei neutralem pH und bilden kein stabiles und parenteral injizierbares Sol aus. Durch Umhüllen der Magnetkerne mit geeigneten Substanzen gelingt es, die Balance der Kräfte zwischen den Partikeln so zu verschieben, daß die thermische Energie (Brownsche Molekularbewegung) der Aggregation und Sedimentation entgegenwirkt und ein stabiles Gel erreicht wird. Eine Reihe von Substanzen, die hierfür infrage kommen, sind patent- und literaturbekannt.

Die Hülle muß für eine stabile Suspension sorgen. Im Rahmen einer galenischen Formulierung sollte eine Hitzesterilisation und/oder ein keimfreies Zubereiten möglich sein. Die Partikel müssen weiterhin einen medizinisch diagnostischen und/oder therapeutischen Nutzen haben, sie müssen zudem pharmakologisch und toxikologisch vertretbar sein. An diesen Hürden scheitert die Mehrzahl der Hüllsubstanzen.

Von den genannten ferrimagnetischen Nanopartikeln sind die mit Dextran umhüllten Eisenoxide in der Entwicklung am weitesten fortgeschritten. Aufgrund aufgetretener Nebeneffekte (u.a. Blutdruckabfall) ist die weitere klinische Prüfung in den USA storniert worden (G.L.Wolf "Current status of MR imaging contrast agents: special report", Radiology 172, 709-710 (1989)). Diese Dextranmagnetite werden derzeit einer eingehenden Kontrolle durch das FDA unterzogen (Diagnostic Imaging, October 1990). Dextranmagnetite sind gegenüber Mischungen mit gängigen Lösungsmitteln instabil, auch ist die galenische Stabilität in physiologischer NaCl-Lösung nicht über längere Zeiträume aufrecht zu erhalten. Die Derivatisierung ist eingeschränkt oder nur unter drastischen Bedingungen möglich. Physiologisch kann sich das allergische Potential der Dextrane störend bemerkbar machen, das gleiche gilt für proteinumhüllte Magnetite.

Der medizinisch-diagnostische Nutzen der umhüllten ferri-/ferromagnetischen (superparamagnetischen) Eisenoxid-Partikel (Stammsubstanz) besteht darin, daß diese nach i.v. Applikation von den phagocytierenden Monocyten und von den Makrophagen des Retikuloendothelialen Systems (RES) des gesunden Gewebes in Milz und Leber aufgenommnen werden, nicht aber von Tumoren und Metastasen. Diese lokal unterschiedliche Aufnahme führt bei üblichen Spin-Echo-Sequenzen (z.B. von 400/25) schon kurz nach parenteraler Applikation zur Auslöschung des Signals im Kernspintomographen-Bild des gesunden Leber-, Milzgewebes, dagegen bilden sich Tumore und Metastasen in Leber und Milz hell auf dunklem Untergrund ab. Ähnliches gilt für Tumore/Metastasen des Lymphsystems.

Sowohl für Liposomen als auch für andere partikuläre "drug carrier" werden heute Anstrengungen unternommen, um die alleinige Aufnahme über mononucleare phagocytierende Zellen (RES) zu umgehen. Dies ist bedeutsam für eine selektive Gewebe/Tumoranreicherung im diagnostischen und therapeutischen Bereich.

Der therapeutische Nutzen der beschriebenen Eisenoxidpartikel liegt sowohl in der Anwendung der Eisenpräparate als Antianämika als auch im "magnetischen Targeting", einer Möglichkeit durch externe Magnetfelder die Eisenoxidpartikel und ihnen anhaftende Substanzen gezielt zum Wirkort zu transportieren. Eine weitere Anwendung der Eisenoxid-Partikel liegt in der rückstoßfreien Sensibilisierung durch γ-Strahlen und in der intrazellulären H-Feld-gekoppelten Hyperthermie. Die resonante Kernabsorption von γ-Quanten mit anschließender Re-Emission bzw. Emission von Auger-Elektronen wird nach dem Entdecker als Mößbauer-Effekt bezeichnet. Die Ausnutzung des Effektes für einen strahlentherapeutischen Nutzen wurde von Mills et al. (Nature 336, 787-789, 1988) beschrieben.
Mills verwendet im Gegensatz zu den hier beschriebenen Eisenoxidsolen molekular gelöste Substanzen, die in Lösung nur einen vernachlässigbaren rückstoßfreien Mößbauer-Effekt und damit resonanzverstärkten strahlentherapeutischen Nutzen zeigen. Die Vorteile der resonanten Kernabsorption von γ-Quanten mit anschließender Re-Emission bzw. Emission von Auger-Elektronen liegen auf der Hand. Die primäre radioaktive Strahlenquelle befindet sich dabei außerhalb des Körpers; der nicht radioaktive Sensor wird in das Tumorgewebe eingebracht. Aufgrund des großen Einfangquerschnitts der resonanten Kernabsorption erfolgt in erster Linie ein Einfang der γ-Quanten durch die nicht radioaktiven Strahlensensoren ohne wesentliche Belastung des umgebenden Gewebes. Diese Strahlensensoren (Antennen) können in das Tumorgewebe (am besten intrazellulär) plaziert werden. Die radioaktive Quelle (Sender) und der Empfänger sind aufeinander abgestimmt und müssen physikalischen und strahlenbiologischen Gesichtspunkten genügen. Auf der Empfängerseite kommen bevorzugt infrage ⁵⁷Fe, ⁹⁹Ru, ¹¹⁹Sn, ¹²¹Sb, ¹²⁷I, ¹⁵¹Eu, ¹⁵⁷Gd, die allesamt nicht radioaktiv sind. Ferrite und Magnetite können ohne wesentliche Beeinflußung der physikalischen Eigenschaften mit den oben erwähnten sogenannten Mößbauer-Isotopen dotiert werden.

Die weitere Anwendung ferromagnetischer Materialien geht davon aus, daß die Magnetmaterialien in Form kleiner Partikel in den Tumor eingeführt werden und von außen über die Ankopplung elektromagnetischer Wechselfelder durch Wirbelstrom-, Hystereseverluste bei der magnetischen Umorientierung aufgeheizt werden. Eine regulierbare Energieaufnahme ist über die Wahl des Curiepunktes (Curietemperatur) anhand der Zusammensetzung der Magnetpartikel möglich (s.z.B. Lilly et al. Radiology 154, 243-244 (1985)). In beiden Fällen sollten zur Energieaufnahme Partikel mit Dimensionen größer als die Einzeldomänen verwendet werden. Hier ist ein Kompromiß zu schließen zwischen der physikalisch notwendigen und der je nach Applikationsform pharmakologisch tolerablen Partikeldimension. Es ist allerdings aus elektronenmikroskopischen Aufnahmen von Gewebeschnitten zu folgern, daß auch nanokristalline Partikel nach intrazellulärer Aufnahme zu größeren Komplexen aggregieren, so daß auch nanokristalline Partikel für diese Anwendung infrage kommen.Zudem sprechen anfängliche Messungen an Phantomen dafür, daß auch physikalische Mechanismen existieren, die zur Aufheizung von Einzeldomain-Partikel führen.

Ferner besteht eine Therapiemöglichkeit darin, ¹⁵⁷Gd in die Ferrite einzubauen und über den großen Einfangquerschnitt des ¹⁵⁷Gd für thermische/epithermische Neutronen zu einer Neutronenaktivierung zu kommen. Wie schon in der oben skizzierten resonanten Kernabsorption durch Photonen (Mößbauer) wird auch hierbei das nicht ¹⁵⁷Gd-enthaltende Gewebe kaum Neutronen aufnehmen und damit nicht belastet werden. Eine Neutronenaufnahme ist primär auf die ¹⁵⁷Gd-enthaltenden Areale konzentriert, so daß - falls eine genügende Tumoranreicherung erzielt wird - nur im Tumor durch Sekundärstrahlung (Auger-Elektronen und Photonen) Strahlenschäden gesetzt werden.

Für die Applikation der Ferrite/Magnetite in der Therapie müssen die Partikel mit den entsprechenden Isotopen dotiert werden. Der Grad der Dotierung der Ferrite/Magnetite ist wie auch die Größe, Ladung, Hydrophobizität und eventuelle Zielfindung dem Therapieziel anzupassen.

Es ist bekannt, daß Makrophagen Eisenoxid-Partikel wie auch andere Fremdstoffe aufnehmen und sich an der Peripherie von Tumoren anreichern. Es gelingt weiterhin, Substanzen über das Anheften an monoklonale Antiköper in bestimmten Tumoren anzureichern. Eine gezielt das Tumorwachstum hemmende Strahlen- und/oder Hyperthermie-Therapie wird damit möglich. Zudem kann über die extrakorporale und/oder intrakorporale Strahlensensibilisierung ein vorteilhafter Beitrag zur Synovektomie geleistet werden. Weiterhin kann schon eine geringfügige Akkumulation der eisenoxid-haltigen Makrophagen in Tumornähe zur Tumordetektion mit hochempfindlichen Magnetfeldsonden (SQUID's) ausgenutzt werden.

Ziel der Erfindung ist es, die beschriebenen Nachteile des bekannten Standes der Technik zu überwinden. Der Erfindung liegt damit die Aufgabe zugrunde, vielfältig modifizierbare und dementsprechend variabel einsetzbare, pharmakologisch und toxikologisch vertretbare magnetische Partikel, die sowohl hitzesterilisiert als auch keimfrei zubereitet werden können, mit synthesetechnisch vertretbaren Aufwand zur Verfügung zu stellen.

Überraschenderweise ist es nun gelungen, den pharmazeutischen Anforderungskatalog für den medizinischen Einsatz durch von Glycosaminoglycan umhüllte Magnetite zu erfüllen.

Gegenstand der Erfindung sind daher nanokristalline magnetische Partikel, bestehend aus einem magnetischen Eisenoxidkern aus Fe₃ O₄, γ-Fe₂ O₃ oder deren Mischungen und einer an diesen Kern chemisorbierten Hülle. Diese zeichnen sich dadurch aus, daß das Hüllmaterial aus natürlichen oder synthetischen Glycosaminoglycanen und/oder deren Derivaten mit Molekulargewichten von 500 Da bis 250.000 Da zusammengesetzt, gegebenenfalls durch geeignete Vernetzungsmittel kovalent vernetzt und/oder durch spezifische Zusatzstoffe modifiziert ist.

Die erfindungsgemäßen Chondroitin-Magnetite sind physiologisch als unbedenklich einzustufen. Chondroitin, das zu den Glycosaminoglycanen zählt (s.u.), ist tierischen oder menschlichen Ursprungs, es kommt ubiquitär im Körper vor und wird medizinisch vielfältig eingesetzt. Der Eisenoxidkern macht - solange er durch das Hüllmaterial gut abgeschirmt ist - keine Schwierigkeit. Nach intrazellulärer Auflösung des Eisenoxidkerns wird das freigesetzte Eisen in den körpereigenen Eisenpool eingeschleust. Chondroitin-Magnetite zeigen nach Hitzesterilisation eine überaus gute Verträglichkeit mit LD₅₀-Werten von 20 mMol/kg (Ratte, Maus), sie führen schon in einer Dosierung von 10 uMol/kg zu einer gut abgrenzbaren Darstellung von Tumoren/Metastasen. Damit wird ein bisher kaum erreichter Sicherheitsabstand von 2000 erzielt. Glycosaminoglycane zeichnen sich weiterhin durch eine hohe hydrolytische Stabilität der glycosidischen Bindung aus.

Das gestellte Anforderungsprofil: einheitlicher Eisenoxidkern, chemische Stabilität des Hüllmaterials bei bekannt positivem Wirkungsspektrum, Herstellung der nanokristallinen magnetischen Partikel unter schonenden Bedingungen, Erweiterung der Synthese nach dem Baukastenprinzip (ausgehend von einem Grundkörper können gezielt aufgabenorientierte Modifikationen angebracht werden), hohe Effizienz der diagnostischen und therapeutischen Wirkung, gute Verträglichkeit, keine nennenswerten Nebenwirkungen, keine notwendigen Zusätze zum Abfangen etwaiger Nebeneffekte, großer Sicherheitsabstand (Faktor 2000 als MR Tumor/Metastasen Diagnostikum), anhaltende galenische Stabilität auch nach Einstellung der Isotonie mit Salzen und nach Hitzesterilisation im Endbehältnis, geringe Anzahl von Abbauprodukten nach längerer Lagerung läßt sich durch die beschriebenen glycosaminoglycanumhüllten magnetischen Eisenoxid-Partikel erfüllen, wohingegen Dextran-Magnetite, Protein-Magnetite, Gummi arabicum-Gelatine-Magnetite oder Magnetite mit synthetischen Hüllmaterialien den gestellten Anforderungskatalog nur partiell erfüllen können. Werden die erfindungsgemäßen Partikel in der Therapie eingesetzt, so kann der magnetische Eisenoxid-Kern mit ⁶Li, ⁵⁷Fe, ⁶¹Ni, Ni, ⁶⁷Zn, Zn, Mn, ⁹⁹Ru, ¹⁰¹Ru, ¹¹³Cd, ¹¹⁹Sn, ¹²¹Sb, ¹²⁷I, ¹⁵¹Eu, ¹⁵⁵Gd, ¹⁵⁶Gd oder ¹⁵⁷Gd dotiert sein.

Besonders bevorzugte Ausführungsformen der erfindungsgemäßen magnetischen Partikel sind dadurch gekennzeichnet,
- daß der Kern aus einem magnetischen Eisenoxid besteht, mit Kerndurchmessern die kleiner als die Dimension magnetischer Einzeldomänen sind und/oder
- daß sie mit den für Injektionslösungen und/oder Enteralia gängigen pharmazeutischen Hilfsstoffen als stabile kolloiddisperse wäßrige Lösungen vorliegen, die 0,2 µm filtierbar und hitzesterilisierbar sind und/oder
- daß der Kern im Falle einer Dotierung bevorzugt mit ⁶Li, ⁵⁷Fe, ⁶¹Ni, ¹⁵¹Eu oder ¹⁵⁷Gd dotiert ist und/oder
- daß die natürlichen oder synthetischen Glycosaminoglycane Chondroitinsulfate, Dermatansulfate, Heparansulfate, Heparin und deren synthetische Analoga oder andere Heparinoide sind und/oder
- daß das Hüllmaterial nach Herstellung der umhüllten magnetischen Eisenoxidpartikel zusätzlich mit in der Bio- und Naturstoffchemie üblichen Vernetzern ("cross-linker") vernetzt ist und/oder
- daß an das Hüllmaterial Mono-, Di-, Tri- und Oligoamine und/oder synthetische und biologische Oligopeptide, Proteine gebunden sind und/oder
- daß an das Hüllmaterial reduziertes oder oxidiertes Glutathion gebunden sowie intrapartikulär reversibel vernetzt ist und/oder
- daß an das Hüllmaterial oberflächenaktive Substanzen gebunden sind und/oder
- daß an das Hüllmaterial Lenkstruktursubstanzen, vorzugsweise Hormone, Cholesterol, Lipide, Etherlipide, Proteine, monoklonale Antikörper, Lectine, Tumorlectine, Adhäsionsproteine, Fusionsproteine, Transportproteine und -einheiten, basische Proteine wie Histone, Interleukine, Lipoproteine, wie z.B. LDL, Glycolipide, Interferone, Tumor-Nekrose-Faktoren, Protein A und Adjuvantien Glycosylreste und allgemein Zuckerreste, die für die Komplement- und Immunerkennung eine Rolle spielen, sowie Ribo-und Desoxyribonucleinsäuren und deren Bruchstücke und Bausteine oder deren Mischungen, gegebenenfalls unter Zusatz von Chemotherapeutika, vorzugsweise Cytostatika gebunden sind und/oder
- daß das Vernetzen, das Anbringen von Lenkstrukturen und/oder oberflächenaktiver Substanzen beliebig kombiniert werden können,
- daß sie von Käfigmolekülen, vorzugsweise bestehend aus Untereinheiten des Clathrins und/oder synthetischen Analoga umschlossen sind.

Gegenstand der Erfindung ist auch ein Verfahren zur Herstellung nanokristalliner magnetischer Partikel, bestehend aus einem magnetischen Eisenoxidkern aus Fe₃ O₄, γ-Fe₂ O₃ oder deren Mischungen und einer an diesen Kern chemisorbierten Hülle. Das Verfahren zeichnet sich dadurch aus, daß die Synthese der Eisenoxidkerne und deren Umhüllung gegebenenfalls unter biomimetischen Bedingungen erfolgt (unter biomimetisch soll dabei der Syntheseablauf unter dem physiologischen Bereich nachempfundenen Bedingungen ph 6-8, T ≤ 37°C, p = 1 bar, wässrige Lösung verstanden werden), gegebenenfalls freie funktionelle Gruppen des Hüllmaterials aktiviert und durch Zugabe von Vernetzungsmitteln die Hülle intrapartikulär vernetzt werden und die vernetzte Hülle durch Zusätze von oberflächenaktiven Stoffen, Lenkstruktursubstanzen - gegebenenfalls unter Zusatz von Chemotherapeutika - sowie gegebenenfalls niedermolekularer Substanzen modifiziert werden.

Besonders bevorzugte Ausführungsformen des erfindungsgemäßen Verfahrens sind dadurch gekennzeichnet,
- daß als Hüllsubstanzen natürliche oder synthetische Glycosaminoglycane oder deren Derivate, vorzugsweise Chondroitinsulfate, Dermatansulfate, Heparansulfate, Heparin und deren synthetische Analoga verwendet werden, und/oder
- daß die funktionellen Gruppen des Hüllmaterials der Magnetitstammlösung mit wasserlöslichen Carbodiimid-Derivaten oder in einem Zwei-Phasen-System mit lipophilen Carbodiimiden aktiviert werden, nachfolgend das aktivierte Magnetit gereinigt und isoliert wird und/oder
- daß der aktivierten Magnetitlösung in der Biochemie und Naturstoffchemie übliche bifunktionelle Vernetzungsmittel und/oder Mono-, Di-, Tri- und Oligoamine, synthetische und biologische Oligopeptide, reduziertes oder oxidiertes Glutathion zugesetzt, die nichtreagierten Edukte schonend durch Dialyse abgetrennt werden und das in seiner Hydrophilie veränderte und/oder vernetzte Magnetit auf die gewünschte Endkonzentration eingestellt wird und/oder
- daß der aktivierten Magnetitlösung oberflächenaktive Substanzen zugesetzt und addiert werden und/oder
- daß der aktivierten Magnetitlösung als Lenkstruktursubstanzen vorzugsweise Hormone, Cholesterol, Lipide, Tumorlectine, Adhäsionsproteine, Fusionsproteine, Transportproteine und-einheiten, basische Proteine wie Histone, Interleukine, Lipoproteine wie z. B. LDL, Glycolipide, Interferone, Tumor-Nekrose-Faktoren, Protein A und Adjuvanzien, Verbindungen, die Zuckerreste enthalten, die für die Komplement- und Immunerkennung eine Rolle spielen sowie Ribo- und Desoxyribonucleinsäuren und deren Bruchstücke und Bausteine und deren Mischungen zugesetzt und addiert werden und/oder
- daß der aktivierten Magnetitlösung Chemotherapeutika zugesetzt oder an das Magnetit addiert werden und/oder
- daß der aktivierten Magnetitlösung niedermolekulare Substanzen, die das physiologische Verteilungsmuster der Partikel beeinflussen, zugesetzt und addiert werden und/oder
- daß der aktivierten Magnetitlösung Clathrin zugesetzt wird.

Die Erfindung betrifft weiterhin diagnostische und/oder therapeutische Mittel, gekennzeichnet durch gegebenenfalls mit ⁶Li, ⁵⁷Fe, ⁶¹Ni, Ni, ⁶⁷Zn, Zn, Mn, ⁹⁹Ru, ¹⁰¹Ru, ¹¹³Cd, ¹¹⁹Sn, ¹²¹Sb, ¹²⁷I, ¹⁵¹Eu, ¹⁵⁵Gd, ¹⁵⁶Gd oder ¹⁵⁷Gd isotopendotierte Eisenoxidkerne aus Fe₃ O₄, γ-Fe₂ O₃ oder deren Mischungen, die von einer bioabbaubaren Hülle aus natürlichen oder synthetischen Glycosaminoglycanen und/oder deren Derivaten mit Molekulargewichten von 500-250 000 Da umgeben sind, wobei gegebenenfalls die Hüllmoleküle durch Vernetzungsmittel vernetzt und durch oberflächenaktive Substanzen, Lenkstruktursubstanzen und niedermolekulare Reste modifiziert sind.

Besonders bevorzugte Ausführungsformen der erfindungsgemäßen Mittel sind dadurch gekennzeichnet,
- daß der Kern im Falle einer Dotierung bevorzugt mit ⁶Li, ⁵⁷Fe, ⁶¹Ni, ¹⁵¹Eu oder ¹⁵⁷Gd dotiert ist und/oder
- daß die natürlichen und synthetischen Glycosaminoglycane Chondroitinsulfate, Dermatansulfate, Heparansulfate, Heparin und deren synthetische Analoga sind, die durch in der Bio- und Naturstoffchemie übliche bifunktionelle Vernetzungsmittel und/oder Mono-, Di-, Tri- und Oligoamine, synthetische und biologische Oligopeptide, reduziertes oder oxidiertes Glutathion vernetzt sind und/oder
- daß an die vernetzte Hülle oberflächenaktive Substanzen gebunden sind und/oder
- daß an die vernetzte Hülle Lenkstruktursubstanzen, vorzugsweise Hormone, Cholesterol Lipide, Etherlipide, Proteine, monoklonale Antikörper, Lectine, Tumorlectine, Adhäsionsproteine, Fusionsproteine, Transportproteine und -einheiten, basische Proteine, wie Histone, Interleukine, Lipoproteine, wie z. B. LDL, Glycolipide, Interferone, Tumor-Nekrose-Faktoren, Protein A und Adjuvanzien, Verbindungen, die Zuckerreste enthalten, die für die Komplement- und Immunerkennung eine Rolle spielen, sowie Ribo- und Desoxyribonucleinsäuren und deren Bruchstücke und Bausteine oder deren Mischungen, gegebenenfalls unter Zusatz von Chemotherapeutika, vorzugsweise Cytostatika, gebunden sind und/oder
- daß Untereinheiten von Clathrin käfigartig die umhüllten Partikel umgeben und/oder
- daß die Magnetpartikel in Liposomen, Chylomikronen, Zellen, Zellorganellen, Bakterien- und Virushüllen eingebaut sowie von Lipiden in Form einer Doppelschicht umgeben werden.

Desweiteren betrifft die Erfindung die Verwendung der diagnostischen und/oder therapeutischen Mittel zur Herstellung von Diagnose- und /oder Therapiemitteln für die Radiotherapie, Hyperthermie, Chemotherapie und MR-Diagnostik sowie als biomagnetische Sonde.

Im Gegensatz zu den nach dem Stand der Technik aufgeführten Partikeln werden nanokristalline magnetische Eisenoxid-Partikel beschrieben, die nach dem Baukastenprinzip aufgebaut sind. Sie können ausgehend von einer Stammsubstanz, die aus einem magnetischen Eisenoxidkern und einer Hülle aus beispielsweise Glycosaminoglycan besteht, nach dem Anwendungszweck in Lösung modifiziert werden.

Diese Modifikation kann durch kovalentes oder nichtkovalentes Addieren, Aktivieren und/oder Vernetzen sowie durch deren Kombination erfolgen. Es hat sich als sinnvoll herausgestellt, das Hüllmaterial in vitro erst zu vernetzen, ehe eine Kopplung von Lenkstrukturen erfolgt. Die Kopplung von Lenkstrukturen ohne vorherige Vernetzung kann zur Destabilisierung der magnetischen Partikel führen. Damit kann die Stammsubstanz durch Lenkstrukturen und Addition von Chemotherapeutika in Richtung auf den Einsatz der tumorselektiven Diagnose/Therapie ergänzt werden. Zudem steht eine Palette von Möglichkeiten zur Verfügung, die Stammsubstanz selbst zu modifizieren. Der Hintergrund dieser Vielfalt liegt in dem breiten Anwendungsspektrum. Es wird unterschieden zwischen der Stammsubstanz, die aufgrund ihres partikulären Charakters vom Retikuloendothelialen System (RES) erkannt und aufgenommen wird und z.B. der MRI-Konstrastierung von Leber-/Milztumoren (Metastasen) dient. Eine Zielfindung auf andere Organe/Gewebe kann nach parenteraler Gabe dadurch erreicht werden, daß die Verweildauer in der Blutbahn verlängert, die Geschwindigkeit der Extravasation erhöht und über angebrachte Lenkstrukturen eine Organ-oder Gewebeselektion ermöglicht wird. Die Aufnahme durch das RES wird als Parallelreaktion zur Reaktion mit dem Zielorgan verstanden und kann durch die Größe, Ladung, Hydrophobie der Oberfläche und durch Vorsättigung des RES beeinflußt werden.

Als chemisorbierte Hüllsubstanzen werden solche bioabbaubaren Substanzen tierischen/menschlichen Ursprungs benutzt, die die Eisenoxohydroxy-Komplexe stabilisieren und eine Synthese der umhüllten Eisenoxide unter biomimetischen Bedingungen zulassen.

Eine Fällung der Eisenoxide im Basischen wird damit umgangen. Die Stammsole können blutisoton z.B. durch Zusatz von Mannitol und NaCl-stabil hergestellt werden.
Die Größe der entstehenden Partikel kann während der Synthese gesteuert z.B. durch Wahl des Chondroitin-Eisen Verhältnis und muß nicht durch Fraktionierungsverfahren auf einen Wert eingestellt werden.
Es werden während der Herstellung keine organischen Lösungsmittel verwendet, die Stabilität der Partikel ist aber auch im Gegensatz zu den Dextranmagnetiten in einigen organischen Lösungsmitteln gewährleistet. Die Substanzen haben vielfältigen diagnostischen und therapeutischen Nutzen. Eine chemische Bindung der Hüllmoleküle an den Eisenoxidkern, wie in der europäischen Patentanmeldung (Publikationsnummer 0284549) beschrieben, wird vermieden, um die biologische Abbaubarkeit zu gewährleisten. Der tierische/menschliche Ursprung des verwendeten Hüllmaterials schlägt sich in der guten Verträglichkeit nieder. Es sind auch "pro-drug"-Formen und Hüllsubstanzen mit pH-abhängigen Bruchstellen herstellbar.

Die Hülle aus Glycosaminoglycan sorgt mit den Carbonsäuregruppen für eine feste Chemisorption auf dem Eisenoxidkern (und mit den Schwefelsäure- und N-Acetylresten für die hinreichende Stabilität des Sols). Der Eisenoxidkern ist dabei soweit abgedeckt, daß im Gegensatz zu den meisten herkömmlichen Formulierungen physiologische Unverträglichkeiten bei parenteraler Applikation hinreichend kompensiert sind.

Als Hüllmaterial kommen wasserlösliche Glycosaminoglycane infrage wie Chondroitinsulfate, Keratansulfate, Dermatansulfate, Heparin, Hyaluronsäure und Heparansulfate und synthetische Analoga. (Zu den vier erwähnten Hauptvertretern der Glycosaminoglycane (Muscopolysaccharidpolyschwefelsäureestern) wird oft auch die Hyaluronsäure hinzugezählt. Die Hyaluronsäure enthält im Gegensatz zu Chondroitinsulfat, Keratansulfat, Dermatansulfat und Heparan (Heparin)-sulfat keine Schwefelsäurereste und ist selbst auch nicht proteinkonjugiert, sie fördert aber die Proteoglycanaggregation.) Glycosaminoglycane bilden zusammen mit den Bindegewebeproteinen die ubiquitär vorkommenden Proteoglycane.

Bei den konventionellen naßchemischen Verfahren der Magnetitherstellung werden saure Fe(II):Fe(III) Lösungen bei erhöhter Temperatur durch Zugabe von Lauge (z. B. NaOH, NH₄OH) auf einen basischen pH eingestellt. Das sich bildende Magnetit fällt in Anwesenheit des Coatungsmittels aus und reißt dieses mit, oder das Coatungsmittel wird nach der Bildung der Magnetite zugesetzt. In einigen Fällen wird während der Magnetitsynthese Ultraschall angelegt, dies führt zur Verkleinerung der entstehenden Partikel (s. Menz et al. WO 9001295). Die nachfolgenden Schritte beinhalten Peptisierung, Neutralisierung, Reinigung, Zugabe von Stabilisatoren, Abfüllen und Hitzesterilisation - wenn möglich - im Endbehältnis.

Im Gegensatz zu diesen Verfahren, die sich geringfügig durch die Ausgangskonzentrationen, Reaktionstemperaturen, Zugabegeschwindigkeiten, Wahl des Coatungsmittels und durch die Aufarbeitungsschritte unterscheiden, gelingt bei den hier beschriebenen Verfahren die Bildung der Glycosaminoglycangecoateten Magnetite im neutralen Milieu (pH 7) und ambienten Temperaturen, auch kann auf eine Ultraschallbehandlung verzichtet werden. Der pH kann in einem Chemostaten konstant im Neutralen gehalten werden. Der basische Bereich, in dem viele Coatungsmittel und biologische Materialien hydrolyseinstabil sind, kann gänzlich vermieden werden, welches sich als großer Vorteil herausstellt, da die Anzahl möglicher Abbauprodukte auf diese Art klein gehalten werden kann.

Die Glycosaminoglycane der erfindungsgemäßen Magnetite sorgen für eine weitgehend abgeschirmte Oberfläche, so daß spezifische Wechselwirkungen minimiert oder vermieden werden. Bei Erythrocyten wird als Folge der Poly-N-acetyl-lactosamine eine weitreichende Hydrathülle diskutiert (J.Viitala and J.Järnefelt, TIBS, Octover 1985, 392-395). Es erscheint wichtig für die Makrophagenaufnahme der Stammsubstanz, daß die Hülle um den Magnetitkern keine spezifischen Reaktionen auslöst, dennoch aber vom Immunsystem aktiviert wird, so daß die opsonierten Partikel von den Makrophagen aufgenommen werden. Für andere Zwecke bieten die Wahl und Derivatisierung der Umhüllung (Ladung, Hydophobizität) neben der Größe eine Möglichkeit, die extrazelluläre Wechselwirkung der Magnetite passiv zu beeinflussen; ein direktes drug targeting ist zudem über das Anheften spezifischer Erkennungsmerkmale (z.B. monoklonale Antikörper, Hormone, Lenkproteine) möglich. Ähnlich wie bei den zahlreichen Liposomenanwendungen sind die Wechselwirkungen von parenteral applizierten Partikeln bisher nur phänomenologisch verstanden und sind ein aktuelles Feld der biochemischen, pharmakologischen und medizinischen Forschung.

Die erfindungsgemäß beschriebenen Magnetitpartikel, die vorzugsweise aus einem magnetischen Eisenoxidkern (ohne oder mit zusätzlicher Dotierung) und einer Hülle aus Glycosaminoglycanen und aus deren Derivaten bzw. synthetischen Analoga bestehen, haben den Vorteil, daß die Hüllmaterialien bioabbaubare Produkte sind. Eisen wird nach intrazellulärer Aufnahme aus dem Eisenoxid freigesetzt und in den Eisenpool (Hämoglobin, Eisenspeicherproteine) eingebaut. Bei einer für die MR Tomographie ausreichenden diagnostischen Dosis von 10-30 µ Mol/kg Körpergewicht wird durch einmalige Applikation der normale Eisenpool eines Erwachsenen nur unwesentlich angehoben. Bei der zugrunde liegenden Synthese lassen sich mit Ausnahme der Auflösung der Eisensalze extreme pH-Bereiche vermeiden, so daß die Hydrolyseanfälligkeit und/oder eine pH-induzierte chemische Modifikation des Hüllmaterials nicht ins Gewicht fallen.

Die in Wasser gelösten Eisenoxidpartikel mit einer Hülle aus Glycosaminoglycan stellen die Stammlösung dar. Die Partikel lassen sich in situ durch Anbringen nieder- und hochmolekularer Substanzen/Liganden/Leitstrukturen und durch Vernetzen sowie Einschließen und Addieren an Zellen oder Zellbestandteilen - wie in den folgenden Beispielen exemplarisch beschrieben - in ihren physikalischen und pharmakologischen Parametern beeinflussen. Damit wird ein Anpassen an spezielle Diagnose- und/oder Therapieprobleme erst möglich. Das Vorgehen ist dabei so ausgewählt, daß die vorgefertigten Magnetite nach Beispiel 1 oder 2 durch Aktivierung der reaktiven Gruppen in eine Form überführt werden, die eine Kopplung mit geeigneten Lignanden/Spacern/Substraten/Lenkstrukturen in physioligisch vertretbarer Form zuläßt.

### Beispiel 1: (konventionelle Synthese)

18.24gChondroitin-4-sulfat (Sigma Typ A) werden unter Wärme in 400 ml destilliertem Wasser gelöst und mit Stickstoff begast.
In 210 ml einer 1 M Fe(III)-chlorid Lösung werden unter Stickstoff 20.6 g Fe(II)-chlorid gelöst. Die frisch zubereitete Fe(II)/Fe(III)-Lösung wird langsam (≈0,5 ml/min) unter Stickstoffspülung zu der 75°C warmen Chondroitinsulfatlösung getropft, so daß sich der an der Eintropfstelle bildende Niederschlag sofort wieder löst. Danach wird langsam zuvor entgaste 3N NaOH-Lösung zugegeben. Es wird bis auf pH 10 titriert. Anschließend wird neutralisiert und 3 h am Rückfluß gekocht, wobei auf pH 7 kontinuierlich nachgeregelt wird. Nach Abkühlen auf Raumtemperatur und Zentrifugation (10 min, 3000 U/min) wird die überstehende Lösung über eine 3 kDa Hollow Fiber Kartusche gegen 10 l destilliertes Wasser diafiltriert und anschließend am Rotationsverdampfer auf ein Volumen von 250 ml eingeengt. Der pH wird auf 7 eingestellt. Nach 0,2 µm Filtration wird die gebrauchsfertige Lösung bei 121°C autoklaviert. Ausbeute 100% bezogen auf das eingesetzte Eisen.

### Beispiel 2: (biomimetische Synthese)

5,0 g Chondroitin-4-sulfat werden in 200 ml Eisen-III-hydroxidsol, entsprechend 6,6 mM Fe (s.Jander/Blasius, Lehrbuch der analytischen und präparativen anorganischen Chemie, S.Hirzel Verlag Stuttgart) gelöst. Eine evtl. auftretende Trübung wird abfiltriert. Zu der auf 37°C erwärmten Lösung erfolgt die tropfenweise Zugabe einer annähernd neutralisierten Lösung von 1,282 g Ammoniumeisen-II-sulfathexahydrat, wobei der langsam ins saure driftende pH durch verdünnte Lauge auf physiologische pH-Werte nachreguliert wird. Das fertige Magnetit wird gereinigt und autoklaviert, wodurch auch die Ausheilung eventueller Fehlstellen der Magnetitkristalle beschleunigt wird.

### Beispiel 3: (Dotierung)

Wie Beispiel 1, nur wird 5% des eingesetzten Fe(II) durch ⁶¹Ni als Mößbauer-aktives Isotop für die Anwendung in der resonanten Kernabsorbtionstherapie ersetzt. Dazu werden 19.57g FeCl₂ und 1.05g ⁶¹NiCl₂ anstelle der 20.6g FeCl₂ verwendet.

### Beispiel 4: (Desulfatierung zur Veränderung der Hydrophilie)

10 ml Chondroitinsulfat-Magnetit nach Beispiel 2 werden über einen Dowex Kationenaustauscher eluiert und mit ca. 7.5 ml Pyridin neutralisiert. Nach Lyophilisieren erhält man das Pyridiniumsalz des Chondroitinsulfat-Magnetits. Das Salz wird in DMSO/Äthanol oder DMSO/H₂0 gelöst und 5 h bei 80°C gehalten. Nach Abkühlen wird mit Wasser verdünnt und mit NaOH pH 9-9.5 eingestellt. Die so eingestellte Dispersion eines desulfatierten Chondroitin-Magnetits wird gegen Wasser dialysiert, ultrafiltriert (10 kDa) und sterilfiltriert.

### Beispiel 5: (Aktivierung der freien Carbonsäuren)

1 ml Chondroitin-4-sulfat Magnetit nach Beispiel 2 wird mit destilliertem Wasser 1:10 verdünnt und der pH mit HCl auf 4.5 eingestellt. Unter Rühren wird in 1,5 fachem Überschuß (bezogen auf die zu aktivierenden funktionellen Gruppen) ein wasserlösliches Carbodiimid (1-Ethyl-3(3-dimethylaminopropyl)-carbodiimid-HCl (EDC.Pierce)) zugegeben und der pH konstant auf 4.5 gehalten (1h bei 4°C). Nach Ende der Reaktion wird das nicht-reagierte Edukt vom aktivierten Magnetit schonend durch Dialyse abgetrennt und das Retentat lyophilisiert.

Sollen mit dem EDC aktivierten Magnetit Folgereaktionen (z.B. Kupplung mit biogenen Liganden) durchgeführt werden, die im pH Bereich 4-5 nicht stabil sind bzw. an biologischer Aktivität verlieren würden, so muß die EDC-Reaktion modifiziert werden. Für die Kopplung/Vernetzung unter physiologischen Bedingungen bzw. im Rahmen einer biomimetischen Synthese muß dem Chondroitin-Magnetit bzw. dem Reaktionsgemisch aus Chondroitin-Magnetit und Ligand oder/und Cross-Linker N-Hydroxysulfosuccinimid (Sulfo-NHS,Pierce) zugesetzt werden, um die Stabilität des Intermediärproduktes bei physiologischen pH Werten zu erhöhen und damit die Ausbeute zu steigern und die biologische Aktivität zu gewährleisten. Im Gegensatz zur EDC Reaktion ohne Sulfo-NHS Zusatz, bei der Intermediär ein O-Acyl-Harnstoffderivat gebildet wird, welches saure Reaktionsbedingungen erfordert und hydrolyseempfindlich ist, entsteht durch den Zusatz von Sulfo-NHS ein stabiles Intermediärprodukt (s.Staros et al., Analytical Biochemistry 156, 220-222, 1986). Das zusätzliche Reagenz hat keinen Einfluß auf das Endprodukt. Es entsteht ebenfalls nach Zugabe eines aminhaltigen Liganden eine Amidbindung und das niedermolekulare Reagenz wird nach der Vermittlung der Kondensation zwischen Säure- und Aminkomponente durch Dialyse der Ultrafiltration aus der kolloidalen Lösung entfernt.

### Beispiel 6: (Desulfatiert EDC)

10 ml desulfatiertes Chondroitin-Magnetit, hergestellt nach Beispiel 4, werden mit EDC wie unter Beispiel 5 umgesetzt. Es resultieren aktivierte Magnetite, die durch Reaktion mit der Säuregruppe des Hüllmaterials je nach EDC Gehalt negativ geladen bis neutral sind.

### Beispiel 7: (Kondensation mit Glucosamin)

Magnetitlösungen nach Beispiel 5 werden mit 1.5 fachem Überschuß Glucosamin versetzt. Durch die Kondensationsreaktion zwischen der EDC-aktivierten Carbonsäure und dem Glucosamin resultiert eine Amidbindung. Das so erhaltene, in seiner Hydrophilie veränderte Magnetit wird gegen destilliertes Wasser dialysiert und auf die gewünschte Konzentration eingestellt.

### Beispiel 8: (Vernetzung mit Ethylendiamin)

Wie Beispiel 7, nur wird anstelle des Glucosamins das bifunktionelle Ethylendiamin zugesetzt. Um ein interpartikuläres Vernetzen zu vermeiden, wird bei niedrigen Magnetitkonzentrationen vernetzt und nach der Vernetzung aufkonzentriert.

### Beispiel 9: (Transferrin-Kopplung)

10 ml Magnetitlösung entsprechend Beispiel 5, pH auf 7.4 eingestellt, werden mit 10 ml (10-20 mg/ml) Human-Transferrin, das zuvor mit einem ca. 10-fachen molaren Überschuß an Eisen (II) in einem Phosphat-Citrat Puffer in das biologisch wirksame Fe-III-Transferrin überführt wurde, versetzt. Bei 4°C wird für 6 Stunden gerührt und der pH auf dem physiologischen pH-Wert gehalten. Anschließend erfolgt bei 4°C eine Ultrafiltration, um das nicht gebundene Transferrin abzutrennen. Das nicht mit Fe-Transferrin vernetzte bzw. beladene Chondroitin-Magnetit und Chondroitin-Magnetit, das mit biologisch nicht aktivem Transferrin beladen ist, werden über eine zuvor mit Anti-H-Transferrin beladene CNBr Sepharose 4 B (Pharmacia) abgetrennt (s.van Ejik and van Noort, J.Clin.Chem.Clin.Biochem. Vol.14, 475-478, 1976).
Abschließend erfolgt eine Gehaltsbestimmung und nach dem Einstellen der gewünschten Endkonzentration eine Sterilfiltration. Ausbeute Eisen: Transferrin = 1:1 bis 5:1 (w/w). Die Lagerung erfolgt bei 4°C, wobei die Stabilität, wie für immobilisierte Proteine bekannt, in Relation zum nativen Transferrin erheblich verbessert ist. Nach dem selben Reaktionsschema lassen sich andere Lenkstrukturen wie monoklonale Antikörper und/oder Cytostatika an das zuvor mit EDC-aktivierte Magnetit (Beispiel 5) koppeln.

### Beispiel 10: (hydrophobe Magnetite)

1g Chondroitin-Magnetit lyphilisiert nach Beispiel 1 wird mit 100 ml DMSO/Aceton aufgenommen und angesäuert. Anschließend wird Dicyclohexylcarbodiimid (5-facher Überschuß bezogen auf die Carbonsäuren) zur Lösung gegeben und mit dem lipophilen Amin Benzylamin in 2-fachen Überschuß bezogen auf die Carbonsäuren versetzt. Es resultiert ein hydrophobes Magnetit. Die Magnetite haben in Ethanol eine Größe von 50 nm (in Wasser 70 nm).

### Beispiel 11: (Lipiddoppelschicht-Magnetite)

Wie Beispiel 7 nur wird als Aminkomponente Phosphatidylethanolamin (Cephalin aus Eigelb) stoichiometrisch - bezogen auf die EDC-Gruppen - unter N₂ zugegeben und 2h bei 4°C gehalten. Das Cephalin verdrängt mit der Ethanolaminkopfgruppe das EDC und bindet an das Chondroitin-Magnetit. Durch Zugabe von Phosphatidylcholin (Lecithin) in 1.5-fachem Überschuß bezogen auf Cephalin bzw. durch ein Gemisch aus Lecithin und Cholesterol wird eine komplette Doppelschicht ausgebildet. Vorteilhaft erweist sich während der Cephalin-Bindung ein Wechsel des Lösungsmittel (EtOH-Wasser-Gemisch). Zur Ausbildung der Doppelschicht muß das Ethanol abgezogen werden.

### Beispiel 12: (Clathrin-Käfige)

Clathrin wird nach bekannten Verfahren in der Monomerform isoliert (s.Review B.M.F. Pearse & R.A. Crother "Structure and Assembly of Coated Vesicles", An. Rev. Biophys. Biophys. Chem. 16, 49-68 (1987)). Nach Zugabe der vorgefertigten Magnetite (s.Beispiel 1,2) im Verhältnis Fe: Protein = 1:1 bis 5:1 (w/w) werden die Clathrin-Monomere zu den typischen Clathrin-Käfigen über eine Absenkung des pH auf 6.2 und Zugabe von MgCl₂ kondensiert. Nicht während der Kondensation eingeschlossene Magnetite und leere Clathrinkäfige werden chromatographisch von den beladenen Käfigen abgetrennt.

### Beispiel 13: (kleine Magnetite)

Die Herstellung des Chondroitinsulfat-Magnetits nach Beispiel 2 wird so abgewandelt, daß die Reaktionslösungen durch eine Filterkartusche umgepumpt werden und ein Teil der Magnetpartikel im Filtrat erscheint; auf diese Weise werden besonders kleine Magnetite hergestellt, die für die i.v. Lymphographie geeignet sind und eine verlängerte Bluthalbwertzeit zeigen.

### Beispiel 14: (Erythrocyteneinschluß)

Erythrocyten werden in üblicher Weise von den anderen Blutbestandteilen getrennt und in physiologischer NaCl-Lösung aufgenommen. Die vorgefertigten Magnetite werden zugesetzt (0.56 mg/ml Fe bei 1mg/ml Protein). Anschließend wird die Lösung gemäß Offenlegung DE 3812816 A1 bei 37°C mit 50bar Lachgas beaufschlagt. Nach Druckentspannung wird das nicht eingeschlossene Magnetit durch Zentrifugation abgetrennt. Man erhält Erythrocytenhüllen, die nach schonendem Verfahren Magnetit aufgenommen haben.

### Abbildungen

### Fig.1:

Kernspintomographie Aufnahme. Axialer Schnitt durch eine Mausleber vor (Baseline), 1, 10 und 30 min nach Applikation von 10 µ Mol/kg eines Chondroitin-Magnetits (Chondroitin-4-sulfat) hergestellt nach Beispiel 1. CSI GE 2T.
Das RES im gesunden Lebergewebe nimmt die Magnetite auf, so daß es im gesunden Lebergewebe zu einer Signalauslöschung kommt. Tumore oder Tumormetastasen haben kein RES, nehmen damit auch kein Magnetit auf und zeigen keine Veränderung der Signalintensität. Die Metastasen sind im Leerbild (Baseline) nicht sichtbar, sie heben sich aber - wie hier gezeigt - schon bei einer Dosis von 10 µ Mol/kg deutlich vom gesunden Lebergewebe ab.

### Fig.2:

Kinetik des Magnetitabbaus in der Rattenleber für ein nach Beispiel 1 hergestelltes Chondroitin-Magnetit (Chondroitin-4-sulfat). Dargestellt ist die relative Intensität (MRI-Lebersignal/MRI-Referenzsignal) in Prozent des Leerwertes nach iv Gabe von 20 µ Mol/kg Chondroitin-Magnetit als Funktion der Zeit.

### Fig.3:

Dosisabhängigkeit. Dargestellt ist die relative Intensität (MRI-Lebersignal/MRI-Referenzsignal) 24h nach iv Gabe der Chondroitin-Magnetite (hergestellt nach Beispiel 1) als Funktion der applizierten Dosis.

### Fig.4:

Röntgendiffraktogramm eines lyophilisierten Transferrin-Magnetit-Konjugates hergestellt über eine Perjodataktivierung eines Chondroitin-4-sulfat-Magnetits und anschließende Kopplung des Transferrins. Die Linien lassen sich kubisch im Sinne einer Spinellstruktur indizieren. Das Röntgendiffraktogramm zeigt, daß die Magnetitstruktur auch nach der Perjodataktivierung erhalten bleibt.

### Fig.5:

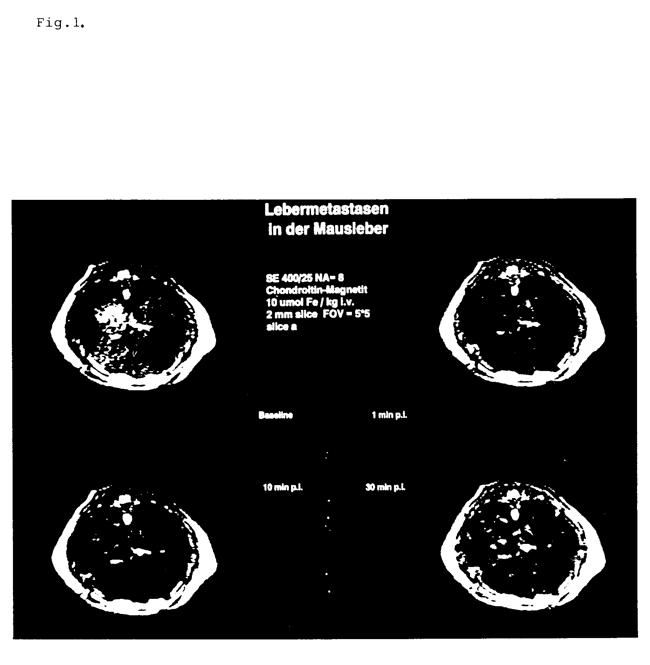

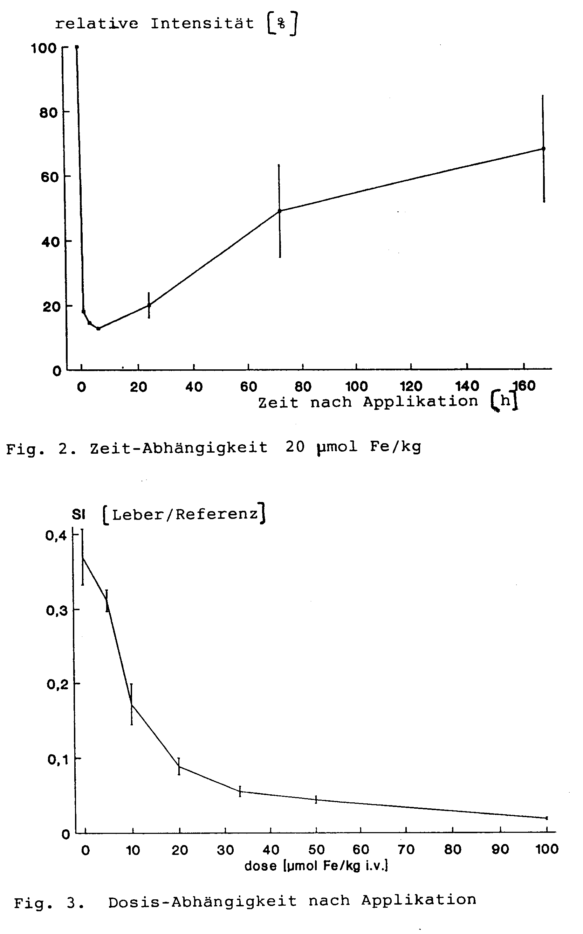

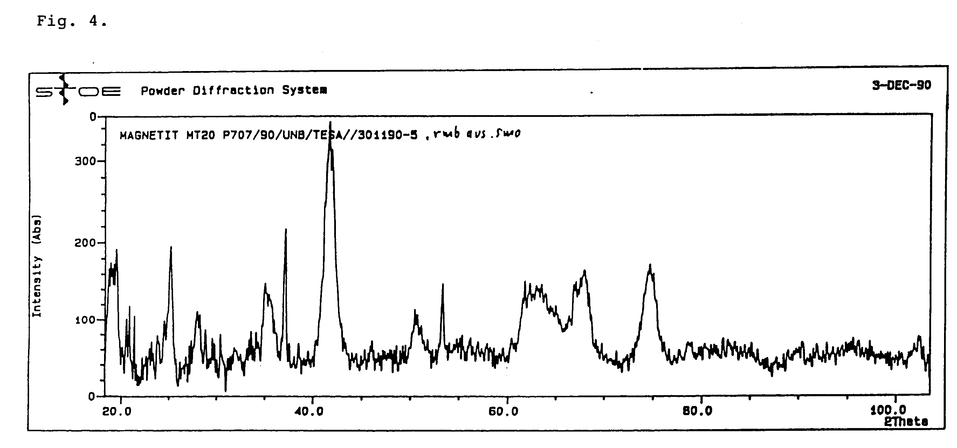

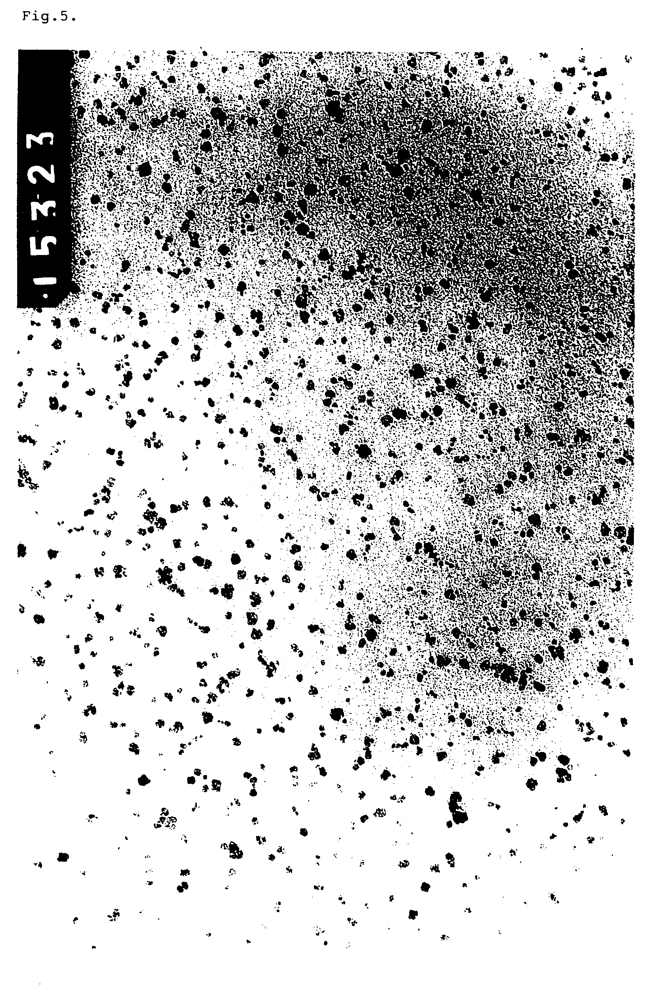

Elektronenmikroskopische Darstellung des Transferrin-Magnetit-Konjugates (s.Fig.4) Vergrößerung der Abbildung 1:300000. Der elektronedierte Magnetitkern wird deutlich sichtbar, es treten keine Aggregate auf.

### Tabelle

**Tabelle 1**

| Daten eines Chondroitin-Magnetits (Chondroitin-4-sulfat) hergestellt nach Beispiel 1 | | |
|---|---|---|
| Größe | | 52 nm (mit Laserlichtstreuung) |
| Relaxivität | T₁ | 40 l/mMol·s |
| | T₂ | 120 l/mMol·s |
| Fe-Konzentration | | 0,33 M |
| akute Verträglichkeit: | | |
| LD₅₀ (Maus) | | 25 mMol/kg |
| Halbwertszeit im Blut: | | 4,4 min |
| (bei einer Dosis von 100 µ Mol/kg Ratte) | | |

## Patentansprüche

1. Nanokristalline magnetische Partikel, bestehend aus einem magnetischen Eisenoxidkern aus Fe₃0₄, γFe₂0₃ oder deren Mischungen und einer an diesen Kern chemisorbierten Hülle, **dadurch gekennzeichnet**, daß das Hüllmaterial aus natürlichen oder synthetischen Glycosaminoglycanen und/oder deren Derivaten mit Molekulargewichten von 500 Da bis 250.000 Da zusammengesetzt, gegebenenfalls durch geeignete Vernetzungsmittel kovalent vernetzt und/oder durch spezifische Zusatzstoffe modifiziert ist.

2. Magnetische Partikel nach Anspruch 1, **dadurch gekennzeichnet**, daß der Kern Durchmesser aufweist, die kleiner als die Dimension magnetischer Einzeldomänen sind.

3. Magnetische Partikel nach Anspruch 1, **dadurch gekennzeichnet**, daß sie gegebenenfalls mit den für Injektionslösungen und/oder Enteralia gängigen pharmazeutischen Hilfsstoffen als stabile kolloiddisperse wäßrige Lösungen vorliegen, die 0.2 µm filtrierbar und gegebenenfalls hitzesterilisierbar sind.

4. Magnetische Partikel nach Anspruch 1 und 2, **dadurch gekennzeichnet**, daß der Eisenoxidkern mit ⁶Li, ⁵⁷Fe, ⁶¹Ni, Ni, ⁶⁷Zn, Zn, Mn, ⁹⁹Ru, ¹⁰¹Ru, ¹¹³Cd, ¹¹⁹Sn, ¹²¹Sb, ¹²⁷I, ¹⁵¹Eu, ¹⁵⁵Gd, ¹⁵⁶Gd oder ¹⁵⁷Gd dotiert ist.

5. Magnetische Partikel nach Anspruch 1, **dadurch gekennzeichnet**, daß die natürlichen oder synthetischen Glycosaminoglycane Chondroitinsulfate, Dermatansulfate, Heparansulfate, Heparin und deren synthetische Analoga oder andere Heparinoide sind.

6. Magnetische Partikel nach Anspruch 1, **dadurch gekennzeichnet**, daß das Hüllmaterial nach Herstellung der umhüllten magnetischen Eisenoxidpartikel zusätzlich mit in der Bio- und Naturstoffchemie üblichen Vernetzern ("cross-linker") vernetzt ist.

7. Magnetische Partikel nach mindestens einem der Ansprüche 1-6, **dadurch gekennzeichnet**, daß an das Hüllmaterial Mono-, Di-, Tri- und Oligoamine und/oder synthetische und biologische Oligopeptide/Proteine gebunden sind.

8. Magnetische Partikel nach mindestens einem der Ansprüche 1-7, **dadurch gekennzeichnet**, daß an das Hüllmaterial reduziertes oder oxidiertes Glutathion gebunden sowie intrapartikulär reversibel vernetzt ist.

9. Magnetische Partikel nach mindestens einem der Ansprüche 1-8, **dadurch gekennzeichnet**, daß an das Hüllmaterial oberflächenaktive Substanzen gebunden sind.

10. Magnetische Partikel nach mindestens einem der Ansprüche 1-8, **dadurch gekennzeichnet**, daß an das Hüllmaterial Lenkstruktursubstanzen, vorzugsweise Hormone, Cholesterol, Lipide, Etherlipide, Proteine, monoklonale Antikörper, Lectine, Tumorlectine, Adhäsionsproteine, Fusionsproteine, Transportproteine und -einheiten, basische Proteine wie Histone, Interleukine, Lipoproteine, wie z.B. LDL, Glycolipide, Interferone, Tumor-Nekrose-Faktoren, Protein A und Adjuvanzien, Glycosylreste und allgemein Zuckerreste, die für die Komplement- und Immunerkennung eine Rolle spielen, sowie Ribo- und Desoxyribonucleinsäuren und deren Bruchstücke und Bausteine oder deren Mischungen, gegebenenfalls unter Zusatz von Chemotherapeutika, vorzugsweise Cytostatika, gebunden sind.

11. Magnetische Partikel nach mindestens einem der Ansprüche 1-10, **dadurch gekennzeichnet**, daß das Vernetzen, das Anbringen von Lenkstrukturen und/oder oberflächenaktiver Substanzen beliebig kombiniert werden können.

12. Magnetische Partikel nach mindestens einem der Ansprüche 1-11, **dadurch gekennzeichnet**, daß sie von Käfigmolekülen, vorzugsweise bestehend aus Untereinheiten des Clathrins und/oder synthetischen Analoga, umschlossen sind.

13. Verfahren zur Herstellung nanokristalliner magnetischer Partikel, bestehend aus einem magnetischen Eisenoxidkern aus Fe₃0₄, γ-Fe₂0₃ oder deren Mischungen und einer an diesen Kern chemisorbierten Hülle, **dadurch gekennzeichnet**, daß die Synthese der Eisenoxidkerne und deren Umhüllung gegebenenfalls unter biomimetischen Bedingungen erfolgen, gegebenenfalls freie funktionelle Gruppen des Hüllmaterials aktiviert und durch Zugabe von Vernetzungsmitteln intrapartikulär vernetzt werden und die vernetzte Hülle durch Zusätze von oberflächenaktiven Stoffen, Lenkstruktursubstanzen - gegebenenfalls unter Zusatz von Chemotherapeutika - sowie gegebenenfalls niedermolekularen Resten modifiziert werden.

14. Verfahren nach Anspruch 13, **dadurch gekennzeichnet**, daß als Hüllsubstanzen natürliche oder synthetische Glycosaminoglycane oder deren Derivate, vorzugsweise Chondroitinsulfate, Dermatansulfate, Heparansulfate, Heparin und deren synthetische Analoga verwendet werden.

15. Verfahren nach Anspruch 13 und 14, **dadurch gekennzeichnet**, daß die funktionellen Gruppen des Hüllmaterials der Magnetitstammlösung mit wasserlöslichen Carbodiimid-Derivaten oder in einem Zwei-Phasen-System mit lipophilen Carbodiimiden aktiviert werden, nachfolgend das aktivierte Magnetit gereinigt und isoliert wird.

16. Verfahren nach Anspruch 13-15, **dadurch gekennzeichnet**, daß der aktivierten Magnetitlösung in der Biochemie und Naturstoffchemie übliche bifunktionelle Vernetzungsmittel und/oder Mono-, Di-, Tri- und Oligoamine, synthetische und biologische Oligopeptide, reduziertes oder oxidiertes Glutathion zugesetzt, die nichtreagierten Edukte schonend durch Dialyse abgetrennt werden und das in seiner Hydrophilie veränderte und/oder vernetzte Magnetit auf die gewünschte Endkonzentration eingestellt wird.

17. Verfahren nach Anspruch 13-16, **dadurch gekennzeichnet**, daß der aktivierten Magnetitlösung oberflächenaktive Substanzen zugesetzt und addiert werden.

18. Verfahren nach Anspruch 13-16, **dadurch gekennzeichnet**, daß der aktivierten Magnetitlösung als Lenkstruktursubstanzen vorzugsweise Hormone, Cholesterol, Lipide, Tumorlectine, Adhäsionsproteine, Fusionsproteine, Transportproteine und -einheiten, basische Proteine wie Hinstone, Interleukine, Lipoproteine wie z.B. LDL, Glycolipide, Interferone, Tumor-Nekrose-Faktoren, Protein A und Adjuvanzien, Glycosylreste und allgemein Zuckerreste, die für die Komplement- und Immunerkennung eine Rolle spielen sowie Ribo- und Desoxyribonucleinsäuren und deren Bruckstücke und Bausteine und deren Mischungen zugesetzt und addiert werden.

19. Verfahren nach Anspruch 18, **dadurch gekennzeichnet**, daß der aktivierten Magnetitlösung Chemotherapeutika zugesetzt oder an das Magnetit addiert werden.

20. Verfahren nach Anspruch 13-19, **dadurch gekennzeichnet**, daß der aktivierten Magnetitlösung niedermolekulare Reste, die das physiologische Verteilungsmuster der Partikel beeinflussen, zugesetzt und addiert werden.

21. Verfahren nach mindestens einem der Ansprüche 13-20, **dadurch gekennzeichnet**, daß der aktivierten Magnetitlösung Clathrin zugesetzt wird.

22. Diagnostische und/oder therapeutische Mittel, gekennzeichnet durch gegebenenfalls isotopendotierte Eisenoxidkerne aus Fe₃0₄, γ-Fe₂0₃ oder deren Mischungen, die von einer bioabbaubaren Hülle aus natürlichen oder synthetischen Glycosaminoglycanen und/oder deren Derivaten mit Molekulargewichten von 500-250 000 Da umgeben sind, wobei gegebenenfalls die Hüllmoleküle durch Vernetzungsmittel vernetzt und durch oberflächenaktive Substanzen, Lenkstruktursubstanzen und niedermolekulare Reste modizifiziert sind.

23. Diagnostische und/oder therapeutische Mittel nach Anspruch 22, **dadurch gekennzeichnet**, daß der Eisenoxidkern mit ⁶Li, ⁵⁷Fe, ⁶¹Ni, Ni, ⁶⁷Zn, Zn, Mn, ⁹⁹Ru, ¹⁰¹Ru, ¹¹³Cd, ¹¹⁹Sn, ¹²¹Sb, ¹²⁷I, ¹⁵¹Eu, ¹⁵⁵Gd, ¹⁵⁶Gd oder ¹⁵⁷Gd dotiert ist.

24. Diagnostische und/oder therapeutische Mittel nach Anspruch 22 und 23, **dadurch gekennzeichnet**, daß die natürlichen und synthetischen Glycosaminoglycane Chondroitinsulfate, Dermatansulfate, Heparansulfate, Heparin und deren synthetische Analoga sind, die durch in der Bio- und Naturstoffchemie übliche bifunktionelle Vernetzungsmittel und/oder Mono-, Di-, Tri- und Oligoamine, synthetische und biologische Oligopeptide, reduziertes oder oxidiertes Glutathion vernetzt sind.

25. Diagnostische und/oder therapeutische Mittel nach Anspruch 24, **dadurch gekennzeichnet**, daß an die vernetzte Hülle oberflächenaktive Substanzen gebunden sind.

26. Diagnostische und/oder therapeutische Mittel nach Anspruch 24, **dadurch gekennzeichnet**, daß an die vernetzte Hülle Lenkstruktursubstanzen, vorzugsweise Hormone, Cholesterol Lipide, Etherlipide, Proteine, monoklonale Antikörper, Lectine, Tumorlectine, Adhäsionsproteine, Fusionsproteine, Transportproteine und -einheiten, basische Proteine, wie Histone, Interleukine, Lipoproteine, wie z.B. LDL, Glycolipide, Interferone, Tumor-Nekrose-Faktoren, Protein A und Adjuvanzien, Glycosylreste und allgemein Zuckerreste, die für die Komplement- und Immunerkennung eine Rolle spielen, sowie Ribo- und Desoxyribonucleinsäuren und deren Bruchstücke und Bausteine oder deren Mischungen, gegebenenfalls unter Zusatz von Chemotherapeutika vorzugsweise Cytostatika, gebunden sind.

27. Diagnostische und/oder therapeutische Mittel nach Anspruch 22-26, **dadurch gekennzeichnet**, daß das Vernetzen, Binden von Lenkstrukturen, oberflächenaktiver Substanzen und Chemotherapeutika im zeitlichen Ablauf beliebig kombiniert werden.

28. Diagnostische und/oder therapeutische Mittel nach mindestens einem der Ansprüche 22-27, **dadurch gekennzeichnet**, daß Untereinheiten von Clathrin und/oder deren Analoga käfigartig die umhüllten Partikel umgeben.

29. Diagnostische und/oder therapeutische Mittel nach mindestens einem der Ansprüche 22-27, **dadurch gekennzeichnet**, daß die Magnetpartikel in Liposomen, Chylomikronen, Zellen, Zellorganellen, Bakterien- und Virushüllen eingebaut sowie von Lipiden in Form einer Doppelschicht umgeben werden.

30. Verwendung der diagnostischen und therapeutischen Mittel nach Anspruch 22-29 zur Herstellung von Diagnose- und Therapiemitteln für die Radiotherapie, Hyperthermie, Chemotherapie und MR-Diagnostik sowie als biomagnetische Sonde.

## Claims

1. Nanocrystalline magnetic particles consisting of a magnetic iron oxide core of Fe₃O₄, gamma-Fe₂O₃ or mixtures thereof and a chemisorbed coating on this core, **characterized by** composition of the coating material of natural or synthetic glycosaminoglycans and/or derivatives thereof with molecular weights of 500 Da to 250,000 Da optionally covalently cross-linked by appropriate cross-linking agents and/or modified by specific additives.

2. Magnetic particles according to claim 1 **characterized by** core diameters smaller than the dimensions of the magnetic single domains.

3. Magnetic particles according to claim 1 **characterized by** the fact that, in case of need, they are available with the common pharmaceutical adjuvants for injectable solutions and/or enteral agents as stable aqueous colloid dispersive solutions which are 0.2 µm filterable and optionally can be heat sterilized.

4. Magnetic particles according to claims 1 and 2 **characterized by** the fact that the iron oxide core is doped with ⁶Li, ⁵⁷Fe, ⁶¹Ni, Ni, ⁶⁷Zn, Zn, Mn, ⁹⁹Ru, ¹⁰¹Ru ¹¹³Cd, ¹¹⁹Sn, ¹²¹Sb, ¹²⁷I, ¹⁵¹Eu, ¹⁵⁵Gd, ¹⁵⁶Gd, or ¹⁵⁷Gd.

5. Magnetic particles according to claim 1 **characterized by** the fact that the natural or synthetic glycosaminoglycans are chondroitin sulfate, dermatin sulfate, heparan sulfate, heparin and synthetic analogues thereof or other heparinoids.

6. Magnetic particles according to claim 1 **characterized by** the fact that the coating material after preparation of enveloped magnetic iron oxide particles, is additionally cross-linked by cross-linkers common in biochemistry and chemistry of natural products.

7. Magnetic particles according to at least one of claims 1-6, **characterized by** the fact that mono-, di-, tri-, and oligoamines and/or synthetic and biological oligopeptides/proteins are bonded to the coating material.

8. Magnetic particles according to at least one of claims 1-7, **characterized by** the fact that reduced or oxidized glutathione is bonded as well as intraparticularly reversibly cross-linked to the coating material.

9. Magnetic particles according to at least one of claims 1-8, **characterized by** the fact that surface-active substances are bonded to the coating material.

10. Magnetic particles according to at least one of claims 1-8, **characterized by** the fact that targetable structure substances, preferably hormones, cholesterol, lipids, ether lipids, proteins, monoclonal antibodies, lectins, tumor lectins, adhesion proteins, fusion proteins, transport proteins and transport units, alkaline proteins such as histones, interleukins, lipoproteins, for example, LDL, glycolipids, interferons, tumor-necrosis factors, protein A and adjuvants, residual glycosyl and general sugar residues which play a role in complement and immune recognition as well as ribonucleic and deoxyribonucleic acids and their fragments and structural elements or mixtures thereof, are bonded to the coating material, if necessary with addition of chemotherapeutic agents, preferably cytostatic agents.

11. Magnetic particles according to at least one of claims 1-10, **characterized by** the fact that the acts of cross-linking, bonding of targetable structures and/or surface-active substances can be combined as desired.

12. Magnetic particles according to at least one of claims, 1-11 **characterized by** the fact that they are surrounded by cage molecules, preferably consisting of subunits of clathrin and/or synthetic analogues.

13. Method for preparation of nanocrystalline magnetic particles consisting of a magnetic iron oxide core of Fe₃O₄, gamma-Fe₂O₃ or mixtures thereof and a coat chemisorbed to the core, **characterized by** synthesis of iron oxide cores and their coating under biomimetic conditions, as the case may be, possibly with free functional groups of the coating material activated and intraparticularly cross-linked by adding a cross-linking agent and the cross-linked coating modified by additives of surface-active substances, targetable structure substances, if necessary, with addition of chemotherapeutic agents and according to need with residual low-molecular weight residues.

14. Method according to claim 13, **characterized by** use of natural or synthetic glycosaminoglycans or their derivatives, preferably chondroitin sulfate, dermatan sulfate, heparan sulfate, heparin and synthetic analogues as coating substance.

15. Method according to claims 13 and 14, **characterized by** the fact that the functional groups of the coating material of the magnetite stock solution are activated by water soluble carbodiimide derivatives or are activated in a two-phase system by lipophilic carbodiimides and that subsequently the activated magnetite is purified and isolated.

16. Method according to claims 13-15**, characterized by** the fact that bifunctional cross-linking agents common in biochemistry and chemistry of natural products and/or mono-, di-, tri-, and oligoamines, synthetic or biological oligopeptides, reduced or oxidized glutathione are added to the activated magnetite solution, with non-reacted educt being gently separated by dialysis and magnetite with its hydrophilia altered and/or cross-linked being adjusted to the desired final concentration.

17. Method according to claims 13-16, **characterized by** the fact that surface-active substances are added and admixed to the activated magnetite solution.

18. Method according to claims 13-16, **characterized by** the fact that targetable structure substances, preferably hormones, cholesterol, lipids, tumor lectins, adhesion proteins, fusion proteins, transport proteins and transport units, alkaline proteins such as histones, interleukins, lipoproteins, for example, LDL, glycolipids, interferons, tumor-necrosis factors, protein A and adjuvants, residual glycosyl and general sugar residues which play a role in complement and immune recognition as well as ribonucleic and deoxyribonucleic acids and their fragments and structural elements as well as mixtures thereof are added to the activated magnetite solution.

19. Method according to claim 18, **characterized by** admixture to the magnetite solution or addition to the magnetite of chemotherapeutic agents.

20. Method according to claims 13-19, **characterized by** admixture and addition to the activated magnetite solution of lower molecular weight residues which influence the physiological distribution pattern of the particles.

21. Method according to at least one of claims 13-20, **characterized by** admixture of clathrin to the activated magnetite solution.

22. Diagnostic and/or therapeutic agents **characterized by**, optionally isotope doped iron oxide cores of Fe₃O₄, gamma-Fe₂O₃ or their mixtures, which are surrounded by a biodegradable coating of natural or synthetic glycosaminoglycans and/or derivatives thereof with molecular weights between 500 - 250,000 Da, with the coating molecules possibly being cross-linked by cross-linking agents and modified by surface-active substances, targetable structure substances and lower molecular weight residues.

23. Diagnostic and/or therapeutic agents according to claim 22, **characterized by** an iron oxide core which is doped with ⁶Li, ⁵⁷Fe, ⁶¹Ni, Ni, ⁶⁷Zn, Zn, Mn, ⁹⁹Ru, ¹⁰¹Ru ¹¹³Cd, ¹¹⁹Sn, ¹²¹Sb, ¹²⁷I, ¹⁵¹Eu, ¹⁵⁵Gd, ¹⁵⁶Gd, or ¹⁵⁷Gd.

24. Diagnostic and/or therapeutic agents according to claims 22 and 23, **characterized by** the fact that the natural or synthetic glycosaminoglycans are chondroitin sulfate, dermatan sulfate, heparan sulfate, heparin and their synthetic analogues which are cross-linked by bifunctional cross-linking agents common in biochemistry and chemistry of natural products and/or are cross-linked with mono-, di-, tri-, and oligoamines, synthetic and biological oligopeptides, reduced or oxidized glutathione.

25. Diagnostic and/or therapeutic agents according to claim 24, **characterized by** bonding of surface-active substances to the cross-linked coating.

26. Diagnostic and/or therapeutic agents according to claim 24, **characterized by** bonding to the cross-linked coating material of targetable structure substances, preferably hormones, cholesterol, lipids, ether lipids, proteins, monoclonal antibodies, lectins, tumor lectins, adhesion proteins, fusion proteins, transport proteins and transport units, alkaline proteins such as histones, interleukins, lipoproteins, for example, LDL, glycolipids, interferons, tumor-necrosis factors, protein A and adjuvants, residual glycosyl and general sugar residues which play a role in complement and immune recognition as well as ribonucleic and deoxyribonucleic acids and their fragments and structural elements or mixtures thereof, if necessary, with addition of chemotherapeutic agents.

27. Diagnostic and/or therapeutic agents according to claims 22-26, **characterized by** the possibility of chronologically variable combination of cross-linking, bonding of targetable structures, surface-active substances and chemotherapeutic agents.

28. Diagnostic and/or therapeutic agents according to at least one of claims 22-27, **characterized by** the fact that the coated particles are surrounded in cage form by subunits of clathrin and/or analogues thereof.

29. Diagnostic and/or therapeutic agents according to at least one of claims 22-27, **characterized by** the fact that the magnetic particles are incorporated in liposomes, chylomicrons, cells, cell organelles, bacteria and virus coatings and are surrounded by lipids in the form of a double layer.

30. Use of the diagnostic and therapeutic agents according to claims 22-29 for preparation of diagnostic and therapeutic agents for radiotherapy, hyperthermia, chemotherapy and MR diagnostics and as biomagnetic probes.

## Revendications

1. Particules nanocristallines magnétiques composées d'un noyau magnétique d'oxyde de fer an Fe₃O₄, en γ-Fe₂O₃ ou en un mélange des deux et d'une enveloppe chimisorbée à ce noyau, caractérisées en ce que le matériau de l'enveloppe est composé de glycosaminoglucanes naturels ou synthétiques et/ou de leurs dérivés de poids moléculaires de 500 Da à 250000 Da, éventuellement réticulés de façon covalente par des agents de réticulation appropriés et/ou modifiés par des additifs spécifiques.

2. Particules magnétiques selon la revendication 1, caractérisées en ce que le noyau présente des diamètres inférieurs à la dimension de domaines magnétiques unitaires.

3. Particules magnétiques selon le revendication 1, caractérisées en ce qu'elles se présentent sous forme de solutions aqueuses stables, en dispension colloïdale, et contiennent les adjuvants pharmaceutiques usuels pour des solutions injectables et/ou parentérales, qui peuvent être filtrées à travers des filtres de 0,2 µm et éventuellement stérilisées à chaud.

4. Particules magnétiques selon les revendications 1 et 2, caractérisées en ce que le noyau d'oxyde de fer est dopé par ⁶Li, ⁵⁷Fe, ⁶¹Ni, Ni, ⁶⁷Zn, Zn, Mn, ⁹⁹Ru, ¹⁰¹Ru, ¹¹³Cd, ¹¹⁹Sn, ¹²¹Sb, ¹²⁷I, ¹⁵¹Eu, ¹⁵⁵Gd, ¹⁵⁶Gd ou ¹⁵⁷Gd.

5. Particules magnétiques salon le revendication 1, caractérisées an ce que les glycosaminoglucanes naturels ou synthétiques sont le sulfate de chondroïtine, le sulfate de dermatane, le sulfate d'héparane, l'héparine et leurs analogues synthétiques ou autres héparinoïdes.

6. Particules magnétiques selon la revendication 1, caractérisées en ce que le matériau de l'enveloppe est en plus réticulé avec les agents de réticulation usuels en biochimie et en chimie des substances naturelles ("cross-linker") après préparation des particules magnétiques d'oxyde de fer et de leur enveloppe.

7. Particules magnétiques selon au moins une des revendications 1 à 6, caractérisées en ce que des mono, di, tri et oligoamines et/ou des oligopeptides/protéines synthétiques et biologiques sont liés au matériau de l'enveloppe.

8. Particules magnétiques selon au moins une des revendications 1 à 7, caractérisées an ce que du glutathion réduit ou oxydé est lié ou réticulé de façon réversible et intraparticulaire ou matériau de l'enveloppe.

9. Particules magnétiques selon au moins une des revendications 1 à 8, caractérisée en ce que des tensioactifs sont liés au matériau de l'enveloppe.

10. Particules magnétiques selon au moins une des revendications 1 à 8, caractérisées en ce que des substances dirigeables de par leur structure, de préférence des hormones, du cholestérol, des lipides, des lipides éther, des protéines, des anticorps monoclonaux, dos lectines, des lectines tumorales, des protéines d'adhésion, des protéines de fusion, des protéines et des unités de transport, des protéines basiques telles que des histones, des interleukines, des lipoprotéines telles que LDL, des glycolipides, des interférons, des facteurs de nécrose tumorale, la protéine A et des adjuvants, des restes glucosidiques et des restes de sucres en général qui jouent un rôle dans la reconnaissance complémentaire et immunitaire, ainsi que des acides ribonucléiques et désoxyribonucléiques et des fragments de ceux-ci et leurs composants ou des mélanges de ceux-ci, en ajoutant éventuellement des agents de chimiothérapie, de préférence des agents cytostatiques, sont liés au matériau de l'enveloppe.

11. Particules magnétiques selon au moins une des revendications 1 à 10, caractérisées en se que la réticulation et la fixation de substances dirigeables de par leur structure et/ou de tensioactifs peuvent être combinées à volonté.

12. Particules magnétiques selon au moins une des revendications 1 à 11, caractérisées en ce que qu'elles sont entourées de molécules formant cages, de préférence composées de sous-unités de la clathrine et/ou d'analogues synthétiques.

13. Procedé pour la préparation de particules magnétiques nanocristallines composées d'un noyau magnétique d'oxyde de fer en Fe₃O₄, en γ-Fe₂O₃ ou en un mélange des deux et d'une enveloppe chimisorbée à ce noyau, caractérisé en ce que la synthèse des noyaux d'oxyde de fer et de leur enveloppe est effectuée éventuellement dans des conditions biomimétiques, des groupes fonctionnels libres du matériau de l'enveloppe sont éventuellement activés et réticulés de façon intraparticulaire par addition d'agents de réticulation, et les enveloppes réticulées sont modifiées par ajout de tensioactifs, de substances dirigeables de par leur structure - éventuellement avec ajout d'agents de chimiothérapie - ainsi qu'éventuellement de groupes à faible poids moléculaire.

14. Procédé selon la revendication 13, caractérisé en ce que l'on utilise des glyoosaminoglucanes naturels ou synthétiques ou leurs dérivés, de préférence le sulfate de chondroïtine, le sulfate de dermatane, le sulfate d'héparane, l'héparine ou leurs analogues syntnétiques comme substances pour l'enveloppe.

15. Procédé selon les revendications 13 et 14, caractérisé en ce que les groupes fonctionnels du matériau de l'enveloppe de la solution mère de magnétite sont activés avec des dérivés cadrodiimides solubles dans l'eau ou dans un système biphasique comprenant des carbodiimides lipohiles, la magnétite activée étant ensuite purifiée et isolée.

16. Procédé salon les revendication 13 à 15, caractérisé en ce que l'on ajoute à la solution de magnétite activée des agents de réticulation bifonctionnels usuels en biochimie et en chimie des substances naturelles et/ou des mono, di, tri et oligoamines dos oligopeptides synthétiques et biologiques, du glutathion réduit ou oxydé, les produits de départ qui n'ont pas réagi sont séparés avec ménagement par dialyse et la concentration finale de la solution de magnétite dont l'hydrophilie a été modifiée et/ou qui a été réticulée est ajustée selon las besoins.

17. Procédé selon les revendications 13 à 16, caractérisé en ce que l'on ajoute à la solution de magnétite activée des tensioactifs que l'on fait réagir.

18. Procédé selon les revendications 13 à 16, caractérisé en ce que l'on ajoute à la solution de magnétite activée dos substances dirigeables de par leur structure, de préférence des hormones, du cholestérol, dos lipides, des lectines tumorales, des protéines d'adhésion, des protéines de fusion, des protéines et des unités de transport, des protéines basiques telles que des histones, des interleukines, des lipoprotéines telles que LDL, des glycolipides, des interférons, des facteurs de nécrose tumorale, la protéine A et des adjuvants, des restes glucosidiques et des restes de sucres en général qui jouent un rôle dans la reconnaissance complémentaire et immunitaire, ainsi que des acides ribonucléiques et désoxyribonucléiques et des parties de ceux-ci et leurs composants ou des mélanges de ceux-ci, que l'on fait réagir.

19. Procédé selon la revendication 18, caractérisé en ce que l'on ajoute des agents de chimiothérapie à la solution de magnétite activée ou on les fait réagir avec la magnétite.

20. Procédé selon les revendications 13 à 19, caractérisé en ce que l'on ajoute à la solution de magnétite activée des restes de faible poids moléculaire que l'on fait réagir et qui influencent le modèle de distribution physiologique des particules.

21. Procédé selon au moins une des revendications 13 à 20, caractérisé en ce que l'on ajoute de la clathrine à la solution de magnétite activée.

22. Agents de diagnostic et/ou thérapeutiques, caractérisés par des noyaux d'oxyde de fer, éventuellement dopés par des isotopes, en Fe₃O₄, en γ-Fe₂O₃ ou en un mélange des deux, entourés d'une enveloppe biodégradable en glycosaminoglucanes naturels ou synthétiques et/ou en leurs dérivés de poids moléculaires de 500 Da à 250000 Da, les molécules de l'enveloppe étant éventuellement réticulées par des agents de réticulation et modifiés par des tensioactifs, des substances dirigeables de par leur structure et des restes de faible poids moléculaire.

23. Agents de diagnostic et/ou thérapeutiques selon la revendication 22, caractérisés en ce que le noyau d'oxyde de fer est dopé par ⁶Li, ⁵⁷Fe, ⁶¹Ni, Ni, ⁶⁷Zn, Zn, Mn, ⁹⁹Ru, ¹⁰¹Ru, ¹¹³Cd, ¹¹⁹Sn, ¹²¹Sb, ¹²⁷I, ¹⁵¹Eu, ¹⁵⁵Gd, ¹⁵⁶Gd ou ¹⁵⁷Gd.

24. Agents de diagnostic et/ou thérapeutiques selon les revendications 22 et 23, caractérisés en ce que les glycosaminoglucanes naturels et synthétiques sont le sulfate de chondroïtine, le sulfate de dermatane, le sulfate d'héparane, l'héparine et leurs analogues synthétiques, réticulés par des agents de réticulation bifonctionnels usuels en biochimie et en chimie des substances naturelles et/ou par des mono, di, tri et oligoamines, par des oligopeptides synthétiques et biologiques, par du glutathion réduit ou oxydé.

25. Agents de diagnostique et/ou thérapeutiques selon la revendication 24, caractérisés an ce que des tensioactifs sont liés à l'enveloppe réticulée.

26. Agents de diagnotic et/ou thérapeutiques selon la revendication 24, caractérisés en ce que des substances dirigeables de par leur structure, de préférence des hormones, du cholestérol, des lipides, des lipides éther, des protéines, des anticorps monoclonaux, des lectines, des lectines tumorales, des protéines d'adhésion, des protéines de fusion, des protéines et des unités de transport, des protéines basiques telles que des histones, des interleukines, des lipoprotéines telles que LDL, des glycolipides, des interférons, des facteurs de nécrose tumorale, la protéine A et des adjuvants, des restes glucosidiques et des restes de sucres en général qui jouent un rôle dans la reconnaissance complémentaire et immunitaire, ainsi que des acides ribonucléiques et désoxyribonucléiques et des fragments de ceux-ci et leurs composants ou des mélanges de ceux-ci, en ajoutant éventuellement des agents de chimiothérapie, de préférence des agents cytostatiques, sont liées à l'enveloppe réticulée.

27. Agents de diagnostic et/ou thérapeutiques selon les revendications 22 à 26, caractérisés en ce que la réticulation et la fixation de substances dirigeables de par leur structure, de tensioactifs et d'agents de chimiothérapie peuvent être combinées à volonté en cours de préparation.

28. Agents de diagnostique et/ou thérapeutiques selon au moins une des revendications 22 à 27, caractérisés en ce que des sous-unités de clathrine et/ou de ses analogues entourent las particules enveloppées d'une sorte de cage.

29. Agents de diagnostic et/ou thérapeutiques selon au moins une des revendications 22 à 27, caractérisés en ce que les particules magnétiques sont introduites dans des liposomes, des chylomicrons, des cellules, des organites cellulaires, des enveloppes de bactéries et de virus et sont entourés de lipides en forme de double couche.

30. Utilisation des agents de diagnostic et thérapeutiques selon les revendications 22 à 29 pour la préparation d'agents de diagnostic et d'agents de thérapie pour la radiothérapie, l'hyperthermie, la chimiothérapie et le diagnostic RM ainsi que comme sonde biomagnétique.
